# EUROPÄISCHE PATENTSCHRIFT

(11) **EP 1 432 828 B1**
(45) Veröffentlichungstag und Bekanntmachung des Hinweises auf die Patenterteilung: **23.04.2008**
(21) Anmeldenummer: 02776809.2
(22) Anmeldetag: 04.10.2002
(51) Int. Cl.: C12Q 1/68

(54) **VERFAHREN ZUM NACHWEIS VON CYTOSIN-METHYLIERUNG IN CPG-INSELN**
METHOD FOR THE DETERMINATION OF CYTOSINE METHYLATION IN CPG ISLANDS
PROCEDE DE DETECTION DE LA CYTOSINE DANS DES ILOTS CPG

(30) Priorität: 05.10.2001 DE 10151055
(43) Veröffentlichungstag der Anmeldung: 30.06.2004
(73) Patentinhaber: Epigenomics AG, 10178 Berlin (DE)
(72) Erfinder: BERLIN, Kurt, 14532 Stahnsdorf (DE)
(74) Vertreter: Schubert, Klemens
(86) Internationale Anmeldenummer: PCT/DE2002/003845
(87) Internationale Veröffentlichungsnummer: WO 2003/031649

(56) Entgegenhaltungen:
- WO-A-01/42493
- WO-A-01/62064
- WO-A-97/46705
- WO-A-99/28498
- DE-A- 10 050 942
- DE-A- 19 951 189
- GONZALGO M L AND JONES P A: "Rapid quantification of methylation differences at specific sites using methylation-sensitive single nucleotide primer extension (Ms-SNuPE)" NUCLEIC ACIDS RESEARCH, OXFORD UNIVERSITY PRESS, SURREY, GB, Bd. 25, Nr. 12, 1997, Seiten 2529-2531, XP002106409 ISSN: 0305-1048 in der Anmeldung erwähnt
- OLEK A ET AL: "A modified an improved method for bisulphite based cytosine methylation analysis" NUCLEIC ACIDS RESEARCH, OXFORD UNIVERSITY PRESS, SURREY, GB, Bd. 24, Nr. 24, 1996, Seiten 5064-5066, XP002106408 ISSN: 0305-1048 in der Anmeldung erwähnt
- XIONG Z AND LAIRD P W: "COBRA: a sensitive and quantitative DNA methylation assay" NUCLEIC ACIDS RESEARCH, OXFORD UNIVERSITY PRESS, SURREY, GB, Bd. 25, Nr. 12, 1997, Seiten 2532-2534, XP002106407 ISSN: 0305-1048 in der Anmeldung erwähnt

## Beschreibung

Die vorliegende Erfindung betrifft ein Verfahren zum besonders empfindlichen Nachweis von Cytosin-Methylierung in DNA-Proben.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußern sich pathogene Zustände in einem veränderten Methylierungsmuster einzelner Gene oder des Genoms.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methyl-cytosin das gleiche Basenpaarungsverhalten aufweist wie Cytosin. Darüber hinaus geht bei einer PCR-Amplifikation die epigenetische Information, welche die 5-Methylcyto-sine tragen, vollständig verloren.

Eine relativ neue und die mittlerweile am häufigsten angewandte Methode zur Untersuchung von DNA auf 5-Methyl-cytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcyto-sin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällung- und Reinigungsschritte durch schnelle Dialyse ersetzt (Olek A, Oswald J, Walter J. A modified and improved method for bisulphate based cytosine methylation analysis. Nucleic Acids Res. 1996 DEC 15;24(24):5064-6). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden: Rein T, DePamphilis ML, Zorbas H. Identifying 5-methylcytosine and related modifications in DNA genomes. Nucleic Acids Res. 1998 May 15;26(10):2255-64.

Die Bisulfit-Technik wird bisher bis auf wenige Ausnahmen (z. B. Zeschnigk M, Lich C, Buiting K, Dörfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) nur in der Forschung angewendet. Immer aber werden kurze, spezifische Stücke eines bekannten Gens nach einer Bisulfit-Behandlung amplifiziert und entweder komplett sequenziert (Olek A, Walter J. The pre-implantation ontogeny of the H19 methylation imprint. Nat Genet. 1997 Nov.;17(3):275-6) oder einzelne Cytosin-Positionen durch eine "Primer-Extension-Reaktion" (Gonzalgo ML, Jones PA. Rapid quantitation of methylation differences at specific sites using methylation-sensitive single nucleotide primer extension (Ms-SNuPE). Nucleic Acids Res. 1997 Jun. 15;25(12):2529-31, WO-Patent 9500669) oder einen Enzymschnitt (Xiong Z, Laird PW. COBRA: a sensitive and quantitative DNA methylation assay. Nucleic Acids Res. 1997 Jun. 15;25(12):2532-4) nachgewiesen. Zudem ist auch der Nachweis durch Hybridisierung beschrieben worden (Olek et al., WO 99/28498).

Harnstoff verbessert die Effizienz der Bisulfit-Behandlung vor der Sequenzierung von 5-Methylcytosin in genomischer DNA (Paulin R, Grigg GW, Davey MW, Piper AA. Urea improves efficiency of bisulphate-mediated sequencing of 5'-methylcytosine in genomic DNA. Nucleic Acids Res. 1998 Nov. 1;26(21):5009-10).

Weitere Publikationen, die sich mit der Anwendung der Bisulfit-Technik zum Methylierungsnachweis bei einzelnen Genen befassen, sind:
Grigg G, Clark S. sequencing 5-methylcytosine residues in genomic DNA. Bioassays. 1994 Jun.;16(6):431-6, 431; Zeschnigk M, Schmitz B, Dittrich B, Buiting K, Horsthemke B, Dörfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol fort bisulphate genomic sequencing. Nucleic Acids Res. 1994 Feb. 25;22(4):695-6; Martin V, Ribieras S, Song-Wang X, Rio MC, Dante R. Genomic sequencing indicates a correlation between DNA hypomethylation in the 5' region of the pS2 gene andin its expression in human breast cancer cell lines. Gene. 1995 May 19;157(1-2):261-4; WO 97 46705, WO 95 15373 und WO 45560.

Ein weiteres bekanntes Verfahren ist die sogenannte methylierungssensitive PCR (Herman JG, Graff JR, Myohanen S, Nelkin BD, Baylin SB. (1996), Methylation-specific PCR: a novel PCR assay for methylation status of CpG islands. Proc Natl Acad Sci U S A. Sep 3;93(18):9821-6). Für dieses Verfahren werden Primer verwendet, die entweder nur an eine Sequenz hybridisieren, die durch die Bisulfit-Behandlung einer an der betreffenden Position unmethylierten DNA entsteht, oder aber umgekehrt Primer, welche nur an eine Nukleinsäure bindet, die durch die Bisulfit-Behandlung einer an der betreffenden Position unmethylierten DNA entsteht. Mit diesen Primer können demnach Amplifikate erzeugt werden, deren Detektion wiederum Hinweise auf das Vorliegen einer methylierten oder unmethylierten Position in der Probe liefern, an welche die Primer binden.

Ein neueres Verfahren ist auch der Nachweis von Cytosin-Methylierung mittels einer Taqman PCR, das als Methyl-Light bekannt geworden ist (WO 00/70090). Mit diesem Verfahren ist es möglich, den Methylierungsstatus einzelner oder weniger Positionen direkt im Verlauf der PCR nachzuweisen, so dass sich eine nachfolgende Analyse der Produkte erübrigt.

Eine Übersicht über den Stand der Technik in der Oligomer Array Herstellung lässt sich aus einer im Januar 1999 erschienenen Sonderausgabe von Nature Genetics (Nature Genetics Supplement, Volume 21, January 1999), der dort zitierten Literatur und dem US-Patent 5994065 über Methoden zur Herstellung von festen Trägern für Zielmoleküle wie Oligonucleotide bei vermindertem nichtspezifischen Hintergrundsignal entnehmen.

Für die Abtastung eines immobilisierten DNA-Arrays sind vielfach fluoresziert markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Matrix-assistierte Laser Desorptions/Ionisations-Massenspektrometrie (MALDI-TOF) ist eine sehr leistungsfähige Entwicklung für die Analyse von Biomolekülen (Karas M, Hillenkamp F. Laser desorption ionization of proteins with molecular masses exceeding 10,000 daltons. Anal Chem. 1988 Oct. 15;60(20):2299-301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI-TOF Spektroskopie eignet sich ausgezeichnet zur Analyse von Peptiden und Proteinen. Die Analyse von Nukleinsäuren ist etwas schwieriger (Gut, I. G. und Beck, S. (1995), DNA and Matrix Assisted Laser Desorption Ionization Mass Spectrometry. Molecular Biology: Current Innovations and Future Trends 1: 147-157.) Für Nukleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. In der MALDI-TOF Spektroskopie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlerweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23: 1367-1373). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis, Molecular Cloning: A Laboratory Manual, 1989.

Nach der Erfindung der PCR sind in den folgenden Jahren zahlreiche Varianten bekannt geworden, die diese Technik zur Amplifikation der DNA verfeinern. Insbesondere ist hier die Multiplexierung der PCR (Multiplex-PCR) zu erwähnen, wobei man mehr als 2 spezifische Primer einsetzt und dabei in einem Reaktionsgefäß eine Vielzahl von verschiedenen, spezifischen Amplifikationen erzeugen kann. Besonders interessant ist auch die sogenannte Nested PCR, welche unter anderem zum Nachweis besonders geringer DNA Mengen verwendet wird. Diese Art der PCR besteht aus zwei aufeinanderfolgenden Amplifikationen, wobei die Primer der zweiten Amplifikation innerhalb des ersten Amplifikates liegen und nicht mit den Primern der ersten Amplifikation identisch sind. Dadurch wird eine besondere Spezifität erreicht, da die Primer der zweiten Amplifikation nur dann funktionieren, wenn in der ersten Amplifikation das beabsichtigte Fragment erzeugt wurde. Dagegen ist die die Vermehrung etwaiger Nebenprodukte der ersten Amplifikation in der zweiten so gut wie ausgeschlossen.

Die WO 01/62064 beschreibt ein Verfahren zur Detektion von 5-Methylcytosin in genomischen DNA-Proben. Dabei wird in einem ersten Schritt genomische DNA aus einer DNA-Probe mit einem Reagenz chemisch umgesetzt, wobei 5-Methylcytosin und Cytosin unterschiedliche reagieren, und anschließend wird die vorbehandelte DNA unter Verwendung einer Polymerase und mindestens einem Primer amplifiziert. Im nächsten Schritt wird die amplifizierte genomische DNA an mindestens ein Oligonukleotid unter Ausbildung einer Duplex hybridisiert und selbiges um mindestens ein Nukleotid verlängert, wobei das Nukleotid eine nachweisbare Markierung trägt und die Verlängerung vom Methylierungsstatus des jeweiligen Cytosins in der genomischen DNA-Probe abhängt. Im nächsten Schritt werden die verlängerten Oligonukleotide auf das Vorhandensein der Markierung untersucht.

Ferner beschreibt die DE 199 51 189 A1 ein Verfahren zur Unterscheidung von 5-Position Methylierungsänderungen von Cytosin-Basen und Cytosin-zu-Thymin Mutationen und zum Nachweis von single nucleotide polymorphisms (SNPs) oder Punktmutation in genomischer DNA.

Es sind demnach bislang vielerlei Verfahren zur Methylierungsanalyse Stand der Technik. Die vorliegende Erfindung soll jedoch eine Möglichkeit zur Analyse des Methylierungsgrades insgesamt in einer CpG Insel bereitstellen. Dabei ist es bevorzugt nicht erforderlich, eine Polymerasereaktion durchzuführen, was die Durchführung des Verfahrens erleichtert. Es ist im Rahmen einer Methylierungsanalyse im Bereich der klinischen Diagnostik wesentlich, dass Untersuchungergebnisse möglichst schnell zur Verfü-gung gestellt werden können und dass sich der experimentelle Aufwand in möglichst engen Grenzen hält. Dazu ist das hier beschriebene Verfahren, welches das Ausmass der Methylierung in einer gesamten CpG Insel misst, in besonderem Masse geeignet. Im Gegensatz zu den meisten bislang beschriebenen Verfahren zur Methylierungsanalyse wird dabei nicht der Methylierunhgsstatus einer einzelnen oder mehrerer einzelner CpG Positionen bestimmt. In vielen Fällen wird dies auch keinen Vorteil bringen, da mitunter ganze Promotorregionen komethyliert vorliegen, das heisst viele aufeinanderfolgende CpG Positionen den gleichen Methylierungsstatus besitzen.

Die vorliegende Erfindung macht es sich nun zunutze, dass dieser Methylierungsstatus in zahlreichen zu untersuchenden Positionen als ähnlich angenommen wird und ein Signal erzeugt werden kann, welches aus der Summe dieser Einzelpositionen resultiert. Dies ermöglicht eine Empfindlichkeit, welche die Durchführung einer PCR Reaktion entbehrlich machen kann.

Auch macht es sich das Verfahren zunutze, dass Guaninbasen in Bisulfit behandelter DNA auf einem Strang nur dann in einer nachfolgenden Polymerasereaktion eingebaut werden, wenn in der entsprechenden genomischen DNA Probe ein methyliertes Cytosin vorlag. Enthält die Proben DNA an den betreffenden Positionen keine Methylierungen, so findet kein Einbau von Guanin in der Polymerasereaktion statt.

Umgekehrt, wenn in einer PCR-Reaktion zu der bisulfitbehandelten DNA auch ein Gegenstrang erzeugt wurde (nach der Bisulfitbehandlung sind die DNA Stränge der Probe nicht mehr wie ursprünglich komplementär), wird mit diesem Gegenstrang als Templat nur dann in einer nachfolgenden Polymerasereaktion ein Cytosin eingebaut, wenn ursprünglich in der entsprechenden genomischen DNA Probe ein methyliertes Cytosin vorlag.

Daraus folgt, dass nur dann Guanine respektive Cytosine eingebaut werden, wenn in der genomischen DNA Probe Methylierung vorlag. Das Ausmass des Einbaus der Guanine respektive Cytosine (je nach Verfahren, siehe oben) korreliert direkt mit dem Ausmass der Methylierung im jeweils untersuchten genomischen DNA Abschnitt.

Das erfindungsgemässe Verfahren besteht daher aus den folgenden Teilschritten:

Zuerst wird aus einer Probe die genomische DNA extrahiert und dabei bevorzugt an eine Oberfläche gebunden, besonders bevorzugt erfolgt diese Bindung durch Hybridisierung an ein immobilisiertes Oligonukleotid und wiederum besonders bevorzugt wird die genomische DNA vor der Bindung durch Restriktionsenzyme gespalten.

Erfindungsgemäß bevorzugt ist es, dass man die Proben DNA aus Serum oder anderen Körperflüssigkeiten eines Individuums gewinnt.

Es ist weiterhin erfindungsgemäß bevorzugt, dass man die Proben DNA aus Zellinien, Blut, Sputum, Stuhl, Urin, Serum, Gehirn-Rückenmarks-Flüssigkeit, in Paraffin einbettetem Gewebe, beispielsweise Gewebe von Darm, Niere, Hirn, Herz, Prostata, Lunge, Augen, Brust oder Leber, histologischen Objektträgern und allen möglichen Kombinationen hiervon gewinnt.

Der enzymatische Schnitt der DNA wird besonders bevorzugt mit einer Restriktionsendonuklease oder mehreren unterschiedlichen Restriktionsenzymen ausgeführt. Werden mehrere Restriktionsendonukleasen verwendet, so hängt es von den jeweiligen Puffern ab, ob diese nacheinander oder gleichzeitig zum Einsatz kommen. Dem Fachmann ist die Anwendung von Restriktionsenzymen gemäß den von den Herstellern mitgelieferten Protokollen bekannt.

Im zweiten Schritt wird eine genomische DNA-Probe bevorzugt mit einem Bisulfit (=Disulfit, Hydrogensulfit) derart behandelt, dass alle nicht methylierten Cytosinbasen in Uracil umgewandelt werden, während die 5-Methylcytosinbasen unverändert bleiben. Dies findet besonders bevorzugt an der Oberfläche statt, an die die Proben DNA im ersten Schritt bereits gebunden wurde.

Es ist ganz besonders erfindungsgemäß bevorzugt, dass man die chemische Behandlung mit einem Bisulfit (=Disulfit, Hydrogensulfit) durchführt. Bevorzugt ist es auch, dass die chemische Behandlung nach Einbetten der DNA in Agarose erfolgt. Es ist auch und weiterhin bevorzugt, dass bei der chemischen Behandlung ein die DNA-Duplex denaturierendes Reagenz und/oder ein Radikalfänger zugegen ist.

Im dritten Verfahrensschritt hybridisiert man an die behandelte DNA ein oder mehrere Oligonukleotide als Primer.

Im vierten Verfahrensschritt wird der oder werden die hybridisierten Primer in einer Polymerasereaktion verlängert. Dabei werden markierte Guaninnukleotide eingesetzt, die im wesentlichen nur dann eingebaut werden, wenn in der behandelten DNA noch Cytosinbasen vorlagen. Demnach ist das Ausmass des Einbaus der Guaninbasen und damit auch die Anzahl der eingebauten Markierungen proportional zu der Methylierung in der zu untersuchenden DNA-Probe. Besonders bevorzugt endet die Polymerasereaktion an der Position, die im ersten Schritt durch den Einsatz einer Restriktionsendonuklease geschnitten wurde.

Im fünften Verfahrensschritt werden die in der Polymeraseraktion nicht eingebauten markierten Nukleotide entfernt. Besonders bevorzugt erfolgt dies durch einfache Waschschritte in dem Fall, dass die DNA an eine Oberfläche gebunden vorliegt.

Im sechsten Schritt des Verfahrens wird die Anzahl der Markierungen in dem durch die Primerverlängerung erzeugten Fragment näherungsweise bestimmt, indem eine von diesen Markierungen ausgehende Signalintensität direkt oder indirekt gemessen wird.

Aus der Signalintensität wird auf den Methylierungsstatus der DNA Probe in dem jeweils untersuchten Fragment geschlossen.

Markierungen können beispielsweise Fluoreszenzmarkierungen, Radionuklide, oder abspaltbare Massenmarkierungen sein, welche in einem Massenspektrometer nachgewiesen werden. Es können jedoch auch Markierungen wie Peptide sein, die indirekt durch Bindung eines wiederum anders markierten Antikörpers nachgewiesen werden. Es sind auch chemische Markierungen denkbar, die durch nachfolgende Umsetzung mit einem wiederum anders markierten Markermolekül, welches zum Beipiel ein Fluoreszenzfarbstoff sein kann, erst sichtbar gemacht werden können. Dem Fachmann sind vielerlei Möglichkeiten geläufig, Moleküle mit Markierungen zu versehen. Die hier aufgeführten Möglichkeiten sollen daher als Beipiele verstanden werden, und andere, dem Fachmann geläufige Möglichkeiten der Markierung sollen als Bestandteil dieser Erfindung betrachtet werden.

In den zuvor genannten Schritten wird auf die Amplifikation der behandelten DNA-Probe verzichtet. Das Verfahren ist daher vor allem dann anwendbar, wenn die Probenmenge nicht limitierend ist und die verwendeten Markierungen mit hinreichender Empfindlichkeit nachweisbar sind. Da jedoch bei der Untersuchung beipielsweise einer CpG Insel nach dem oben beschriebenen Verfahren eine Vielzahl von Markierungen in der Polymerasereaktion eingebaut wird, wenn die CpG Insel methyliert vorlag, wird auch durch die Art des durchgeführten Verfahrens eine erhebliche Empfindlichkeitssteigerung erreicht.

Wird einer erhöhte Empfindlichkeit aufgrund einer nur geringfügigen DNA-Menge benötigt, so wird das oben beschriebene Verfahren durch eine PCR Reaktion oder eine andere nicht nur linear amplifizierende Polymerasereaktion ergänzt, welche nach der Behandlung gemäss dem zweiten Verfahrensschritt durchgeführt wird. Bevorzugt wird dabei die (chemisch) behandelte DNA-Probe unter Verwendung von bevorzugt mindestens 2 Primeroligonukleotiden mittels einer Polymerasereaktion amplifiziert, wobei bevorzugt eine hitzebeständige Polymerase, Nukleotide sowie ein geeigneter Reaktionspuffer, wie meist mit der Polymerase geliefert und dem Fachmann bekannt, verwendet werden.

Besonders bevorzugt ist es auch, die Amplifikationen mehrerer unterschiedlicher Fragmente mit mehr als 2 verschiedenen Primern in einem Reaktionsgefäß durchzuführen und damit die Amplifikationsschritte als Multiplex-PCR auszuführen. Es ist generell besonders bevorzugt, die Amplifikationen als Polymerasekettenreaktion auszuführen.

Soll weiterhin, auch unter Verwendung einer zusätzlichen PCR, an einer Oberfläche gearbeitet werden, so erfolgt entweder eine Festphasen-PCR in der Art, dass Primer für den PCR Schritt zusätzlich an die Oberfläche gebunden sind, oder es erfolgt nach der PCR bevorzugt ein Aufreinigungsschritt durch handelsübliche Aufreinigungskits (wie beipielsweise von den Firmen Promega oder Qiagen) und nachfolgende Bindung des PCR Produktes an eine Oberfläche, an welcher nun die weitere Polymerasereaktion zum Einbau der Markierungen erfolgen soll.

Bevorzugt ist es, dass bei der Amplifikation einer der Primer an eine Festphase gebunden ist. Bei diesen Festphasen kann es sich beispielsweise um funktionalisierte Polymere, Metalle, Glas oder Halbleiter wie Silicium handeln. Das Anbinden der Primer erfolgt bevorzugt über bifunktionale Linkermoleküle, welche an eine silanisierte Oberfläche gebunden werden oder aber beispielsweise über Thioate im Primer oder Thiolmodifikationen an Bromacetylderivatisierte Oberflächen oder Gold.

In einer weiteren, bevorzugten alternativen Variante des Verfahrens erfolgt der Einbau der Markierungen selbst in einer PCR Reaktion. In diesem Fall findet die Reaktion bevorzugt ohne Bindung der Primer an eine Festphase statt. Stattdessen erfolgt eine Abtrennung des PCR Produktes, beipielsweise durch Gelelektrophorese, von anderen Nebenprodukten und Edukten. Die Intensität der von den Markierungen ausgehenden Signale in den so erhaltenen Banden oder Bandenmustern wird bestimmt und so auf den Methylierungsgrad in dem untersuchten Fragment der Proben DNA geschlossen.

Wird auch in einer Amplifikation der komplementäre Gegenstrang zu den behandelten DNA Fragmenten erzeugt, so entsprechen in diesem Gegenstrang im Falle einer Behandlung mit einem Bisulfit Adenin einer nicht methylierten Cytosinposition und Guanin einer methylierten Cytosinposition in der DNA-Probe. Daher ist es nach Amplifikation auch möglich, das Verfahren sinngemäß auch mit einem markierten Cytosin auszuführen.

Die in den Polymerasereaktionen verwendeten Primer amplifizieren besonders bevorzugt keine Fragmente aus nicht mit Bisulfit behandelter genomischer DNA (oder nur in vernachlässigbarem Ausmaß), so dass sie für die mit Bisulfit umgewandelte DNA spezifisch sind. Dies schützt vor fehlerhaften Ergebnissen im Falle einer unvollständigen Umwandlungsreaktion mit beispielsweise Natriumbisulfit.

Bevorzugt ist es ferner, dass die Analyse mittels Hybridisierung an Oligomer-Arrays erfolgt, wobei Oligomere Nukleinsäuren oder in ihren Hybridisierungseigenschaften ähnliche Moleküle wie PNAs sein können.

Es ist bevorzugt, dass der Methylierungsstatus von mehr als 10 Methylierungspositionen der zu analysierenden DNA in einem Experiment nachgewiesen wird.

Auch ist es erfindungsgemäß bevorzugt, dass die Analyse durch zusätzliche Längenmessung der amplifizierten zu untersuchenden DNA erfolgt, wobei Methoden zur Längenmessung Gelelektrophorese, Kapillargelelektrophorese, Chromatographie (z.B. HPLC), Massenspektrometrie und andere geeignete Methoden umfassen. Die Fragmente werden dabei über die in der Polymerasereaktion eingebauten Markierungen detektiert.

Weiterhin ist bevorzugt, dass man aus dem Methylierungsgrad an einzelnen oder mehreren verschiedenen untersuchten CpG Inseln auf das Vorliegen einer Erkankung oder eines anderen medizinischen Zustandes des Patienten schließt.

Bevorzugt werden die Markierungen entweder durch eine Markierung der Nukleotide während der Polymerasereaktion oder Amplifikation in die erzeugten markierten Fragmente eingebracht.

Weiterhin besonders vorteilhaft ist es, dass die Markierungen Fluoreszenzmarkierungen sind oder/und dass die Markierungen Radionuklide sind oder/und dass die Markierungen ablösbare Massenmarkierungen sind, die in einem Massenspektrometer nachgewiesen werden.

Erfindungsgemäß bevorzugt ist es auch, dass die Fragmente insgesamt im Massenspektrometer nachgewiesen werden und somit durch ihre Masse eindeutig charakterisiert sind. Dabei trägt jede eingebaute Markierung eine für die Methylierung spezifisch zusätzliche Masse bei, so dass aus der gemessenen Molekülmasse auf die Anzahl der Methylierungen in der CpG Insel geschlossen werden kann.

Ein weiterer Gegenstand der vorliegenden Erfindung ist auch die Verwendung eines erfindungsgemäßen Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Vorteilhaft ist dabei die Verwendung eines erfindungsgemäßen Verfahrens zur Unterscheidung von Zelltypen oder Geweben oder zur Untersuchung der Zelldifferenzierung.

Gegenstand der vorliegenden Erfindung ist auch ein Kit, bestehend aus einem Bisulfit enthaltenen Reagenz, Primern fü die Polymerasereaktion, sowie optional einer Anleitung zur Durchführung eines erfindungsgemäßen Assays. Zusätzlich ist auch eine Mikrotiterplatte, die für die Immobilisierung der Proben-DNA eine aktivierte Oberfläche besitzt und in der auch nachfolgende Reaktionsschritte ausgeführt werden können, bevorzugter Bestandteil dieses Kits.

Das erfindungsgemäße Verfahren besteht demnach aus den folgenden Teilschritten:
a) aus einer Probe wird DNA extrahiert,
b) die DNA wird, bevorzugt mit einem Bisulfit (=Disulfit, Hydrogensulfit), derart behandelt, dass Cytosin in eine vom Basenpaarungsverhalten in der DNA-Duplex her unterschiedliche Base umgewandelt wird, während 5-Methylcytosin unverändert bleibt,
c) ein oder mehrere Oligonukleotidprimer werden an die behandelte DNA hybridisiert,
d) die hybridisierten Primer werden in einer Polymeraseraktion verlängert, wobei markierte Nukleotide im wesentlichen nur dann eingebaut werden, wenn in der behandelten DNA nach Schritt b)noch Cytosinbasen vorlagen und wobei das Ausmass des Einbaus der markierten Nukleotide zu der Methylierung in der zu untersuchenden DNA-Probe korreliert,
e) die in der Polymeraseraktion nicht eingebauten markierten Nukleotide werden entfernt,
f) die Anzahl der Markierungen in dem durch die Primerverlängerung erzeugten Fragment wird näherungsweise bestimmt, indem eine von diesen Markierungen ausgehende Signalintensität gemessen wird.

Besonders bevorzugt ist es, dass man die Proben DNA aus Serum oder anderen Körperflüssigkeiten eines Individuums gewinnt.

Besonders bevorzugt ist es, dass man die Proben DNA aus Zelllinien, Blut, Sputum, Stuhl, Urin, Serum, Gehirn-Rückenmarks-Flüssigkeit, in Paraffin eingebettetem Gewebe, beispielsweise Gewebe von Augen, Darm, Niere, Hirn, Herz, Prostata, Lunge, Brust oder Leber, histologischen Objektträgern und allen möglichen Kombinationen hiervon gewinnt.

Es ist zudem besonders bevorzugt, dass man die Behandlung nach Anspruch 1b) mit einem Bisulfit (=Disulfit, Hydrogensulfit) durchführt. Besonders bevorzugt ist auch, dass die chemische Behandlung nach Einbetten der DNA in Agarose oder besonders bevorzugt nach Bindung der DNA an eine Oberfläche erfolgt. In einer weiteren besonders bevorzugten Verfahrensvariante ist bei der chemischen Behandlung ein die DNA-Duplex denaturierendes Reagenz und/oder ein Radikalfänger zugegen.

Es ist zudem besonders bevorzugt, dass aus einer Probe genomische DNA extrahiert wird und dabei an eine Oberfläche gebunden wird. Besonders bevorzugt ist es auch, dass diese Bindung durch Hybridisierung an ein immobilisiertes Oligonukleotid erfolgt. Es ist zudem bevorzugt, dass die extrahierte DNA vor der Bindung durch Restriktionsenzyme gespalten wird.

In einer besonders bevorzugten Verfahrensausführung hybridisiert man an die behandelte DNA mehrere verschiedene Oligonukleotide als Primer.

Besonders bevorzugt werden die markierten Nukleotide Guaninderivate sind, wobei diese in der Polymerasereaktion nur dann wesentlich eingebaut, wenn in der DNA Probe an den entprechenden Positionen Cytosin-Methylierung vorlag. Zudem ist bevorzugt, dass das Ausmaß des Einbaus der Guaninbasen und damit auch die Anzahl der eingebauten Markierungen proportional ist zu der Methylierung in der zu untersuchenden DNA-Probe.

In einer besonders bevorzugten Verfahrensvariante endet Polymerasereaktion bevorzugt an der Position, die vor der DNA Isolierung durch den Einsatz einer Restriktionsendonuklease geschnitten wurde.

In einer weiteren bevorzugten Verfahrensvariante werden die in der Polymeraseraktion nicht eingebauten markierten Nukleotide durch Waschschritte entfernt und die DNA liegt an eine Oberfläche gebunden vor.

Die Markierungen sind bevorzugt Fluoreszenzmarkierungen, Radionuklide, Chemiluminisznezmarkierungen oder abspaltbare Massenmarkierungen sind, welche in einem Massenspektrometer nachgewiesen werden. Es ist auch bevorzugt, dass die Markierungen indirekt durch Bindung eines wiederum anders markierten Antikörpers nachgewiesen werden.

In einer weiteren besonders bevorzugten Variante des Verfahrens wird die behandelte DNA-Probe unter Verwendung von bevorzugt mindestens zwei Primeroligo-nukleotiden vorzugsweise mittels einer Polymerasekettenreaktion amplifiziert. In einer weiteren besonders bevorzugten Verfahrensvariante sind die markierten Nukleotide Cytosinderivate, wobei diese in der Polymerasereaktion nur dann wesentlich eingebaut werden, wenn in der DNA Probe an den entprechenden Positionen Cytosin-Methylierung vorlag.

Bevorzugt ist auch ein Verfahren, bei dem man die Amplifikation mehrerer Fragmente in einem Reaktionsgefäß in Form einer Multiplex-PCR durchführt.

In einer weiteren besonders bevorzugten Variante des Verfahrens wird mindestens einer der Amplifikationen einer der jeweiligen Primer an eine Festphase gebunden.

Verfahren nach einem der voranstehenden, dadurch gekennzeichnet, dass die Amplifikate insgesamt im Massenspektrometer nachgewiesen werden und somit durch ihre Masse eindeutig charakterisiert sind.

Gegenstand der vorliegenden Erfindung ist auch die Verwendung eines der beschriebenen Verfahren zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Ebenfalls Erfindungsgegenstand ist die Verwendung eines Verfahrens nach einem der voranstehenden Ansprüche zur Unterscheidung von Zelltypen oder Geweben oder zur Untersuchung der Zelldifferenzierung.

### Kurzbeschreibung der Figuren

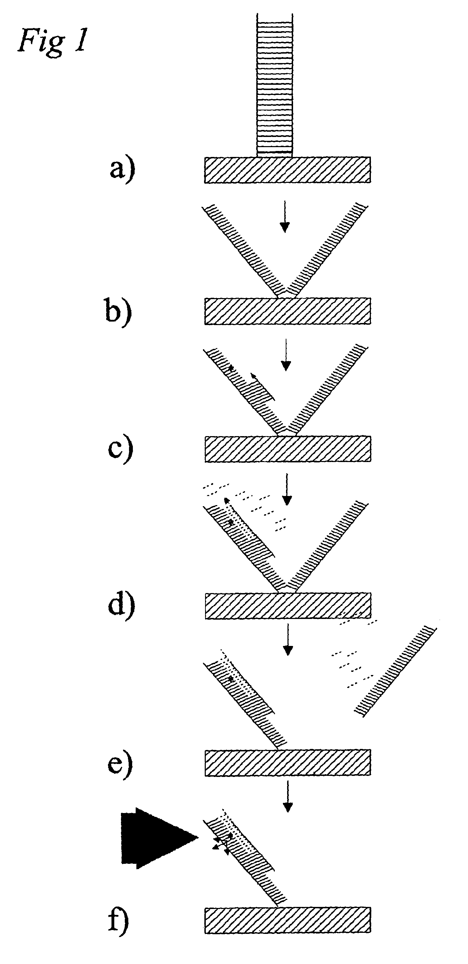

Fig. 1 erläutert ein besonders bevorzugte Variante der Erfindung:
a) enzymatisch geschnittene Proben-DNA wird an eine Oberfläche gebunden und dabei von Begleitmaterial abgetrennt
b) die an die Oberfläche gebundene DNA wird denaturiert und anschliessend beipielsweise mit einem Bisulfit chemisch behandelt
c) ein Primer wird an die DNA gebunden
d) eine enzymatische Primerextensionsreaktion (Nukleotide dargestellt als gestrichelte Linien: ---)wird durchgeführt, und es werden nur dann Markierungen eingebaut, wenn zuvor in der Proben-DNA an den betreffenden Positionen Cytosin-Methylierungen (*)vorlagen
e) die übrigen markierten Nukleotide und Reaktionskomponenten werden in einem Waschschritt entfernt
f) die Fluoreszenzintensität ausgehend von den eingebauten Markierungen wird gemessen.

Die folgenden Beispiele erläutern die Erfindung näher.

### Beispiel 1:

Bisulfit-Behandlung von DNA an festen Oberflächen Im folgenden werden zwei Verfahren beschrieben, um DNA an einer festen Phase zu binden:
a) Verwenden von Reaktionsgefäßen
Für die Anbindung von DNA auf die Oberfläche der Reaktionsgefäße wurde EcoR1 geschnittene genomische DNA (Promega) verwendet. 160ng wurden in die entsprechenden Reaktionsgefäße pipettiert, mit Wasser auf ein Gesamtvolumen von 20µl aufgefüllt, auf einem Shaker kurz gemischt und für 15 Minuten bei Raumtemperatur inkubiert. Anschließend wurde die Lösung entfernt und das Gefäße zweimal mit 50µl Wasser gewaschen. Um die Aktivität der verbleibenden Bindungsstellen auf der Tubeoberfläche herabzusetzen, wurden 10µl einer 5%igen Bovin-Serum-Albumin Lösung hinzu pipettiert, mit 40µl Wasser aufgefüllt und ebenfalls bei Raumtemperatur für 15 Minuten inkubiert. Abschließend wurde das Gefäße einmal mit 50µl Wasser gewaschen.

### Bisulphit Behandlung:

Die angebundene DNA wird bei 96°C für 20 Minuten in einem Eppendorf-Mastercycler denaturiert. Für die Bisulfitreaktion wurde eine Natriumbisulfit-Lösung verwendet. Die Reaktionsgefäße wurden bei 50°C in einem Eppendorf-Mastercycler für fünf Stunden inkubiert. Nach erfolgter Bisulfitreaktion wurde die Lösung herauspipettiert und die Gefäße mit Wasser und, vorbereitend auf die Desulfonierung, mit einer 50mM Tris-HCl-Lösung gewaschen. Die Desulfonierung erfolgt mit 50µl einer 50mM Tris-HCl-Lösung bei pH9 für 20 Minuten bei 96°C. Nach dreimaligem Waschen mit je 50µl Wasser sind die Reaktionsgefäße einsatzbereit für eine Amplifikation mittels PCR.

### b) Verwenden von Polylysin Slides

Die Slides (Objektträger) werden zusammen mit den anzubringenden DNA Molekülen für 3 Minuten bei 42°C in wässrigem Medium inkubiert. Dabei werden die DNA Moleküle gleichmäßig auf der Slide-Oberfläche verteilt. Danach werden die Slides 10 Sekunden auf einer Heizplatte bei einer Temperatur von 150°C inkubiert, wodurch die DNA an die Oberfläche gebunden wird. Um nicht gebundene DNA zu entfernen werden die Slides dann für 5 Minuten mit NH4OH gewaschen. Zum Denaturieren werden die Slides anschließend für 1 Minute bei 95°C (H2O) inkubiert und danach sofort in 95% EtOH überführt wonach sie getrocknet werden.

### Bisulphit Behandlung:

Die angebundene DNA wird bei 96°C für 20 Minuten unter Verwendung eines Adapters in einem Eppendorf-Mastercycler denaturiert. Für die Bisulfitreaktion wurde eine Natriumbisulfit-Lösung verwendet. Die Slides wurden bei 50°C in einem Eppendorf-Mastercycler für fünf Stunden inkubiert. Nach erfolgter Bisulfitreaktion wurden Slides mit Wasser und, vorbereitend auf die Desulfonierung, mit einer 50mM Tris-HCl-Lösung gewaschen. Die Desulfonierung erfolgt mit 50µl einer 50mM Tris-HCl-Lösung bei pH9 für 20 Minuten bei 96°C. Nach dreimaligem Waschen mit je 50µl Wasser sind die Reaktionsgefäße einsatzbereit für eine Amplifikation mittels PCR.

### Beispiel 2:

### Primer Verlängerung (extension) unter Verwendung der DNA des Gens p15

Die an einer festen Phase gebundene DNA des Gens p15 (Accession Nummer NM_004936) diente, nachdem sie wie oben beschrieben mit Bisulphit behandt wurde, als Ausgangsmaterial für die Primer Verlängerung. Für die Primer Verlängerung wurden 2pmol des Primers mit der Sequenz GTTTAGGTTTTTTAGGAAGGAGAGAGTG (SEQ-ID 1) eingesetzt. Lag an der zu verlängernden DNA an der betreffen Stelle ein Guanin vor, so wurde bei dem verlängerten Primer ein markiertes Cytosin eingebaut (methylierter Zustand); lag dagegen ein Adenin vor, so wurde ein (verschieden) markiertes Thymin eingebaut (nicht methylierter Zustand). Gemäß Herstellerangaben (Amersham-Pharmacia) wurde ein Dideoxynukleotidgemisch mit jeweils verschieden markierten Nukleotiden für die Extension verwendet. Die Bedingungen waren: 96°C, 10sec; 55°C, 5sec und 60°C, 10sec. Es wurden 25 Zyklen durchgeführt. Für die Polylysin Slides erfolgte der Nachweis der verlängerten Sequenz mit dem Scanner Axxon 4000A (Axxon Instruments) unter Benutzung der Software GenePix Pro V. 3.0. Die Durchführung der Primer Verlängerung im Reaktionsgefäß erfolgte analog.

## Patentansprüche

1. Verfahren zur Analyse des Methylierungsgrades innerhalb einer CpG-Insel, in DNA-Proben, **dadurch gekennzeichnet, dass** die folgenden Verfahrensschritte ausgeführt werden:
a) aus einer Probe wird DNA extrahiert,
b) die DNA wird, bevorzugt mit einem Bisulfit (=Disulfit, Hydrogensulfit), derart behandelt, dass Cytosin in eine vom Basenpaarungsverhalten in der DNA-Duplex her unterschiedliche Base umgewandelt wird, während 5-Methylcytosin unverändert bleibt,
c) ein oder mehrere Oligonukleotidprimer werden an die behandelte DNA hybridisiert,
d) die hybridisierten Primer werden in einer Polymerasereaktion verlängert, wobei markierte Nukleotide im wesentlichen nur dann eingebaut werden, wenn in der behandelten DNA nach Schritt b) noch Cytosinbasen vorlagen und wobei der Methylierungsstatus in den zu untersuchenden Positionen als ähnlich angenommen wird und wobei das Ausmass des Einbaus der markierten Nukleotide zu der Methylierung in der zu untersuchenden DNA-Probe korreliert,
e) die in der Polymeraseraktion nicht eingebauten markierten Nukleotide werden entfernt,
f) die Anzahl der Markierungen in dem durch die Primerverlängerung erzeugten Fragment wird näherungsweise bestimmt, indem eine von diesen Markierungen ausgehende Signalintensität gemessen wird.

2. Verfahren nach Anspruch 1, **dadurch gekennzeichnet, dass** man die Proben DNA aus Serum oder anderen Körperflüssigkeiten eines Individuums gewinnt.

3. Verfahren nach Anspruch 1, **dadurch gekennzeichnet, dass** man die Proben DNA aus Zelllinien, Blut, Sputum, Stuhl, Urin, Serum, Gehirn-Rückenmarks-Flüssigkeit, in Paraffin eingebettetem Gewebe, beispielsweise Gewebe von Augen, Darm, Niere, Hirn, Herz, Prostata, Lunge, Brust oder Leber, histologischen Objektträgern und allen möglichen Kombinationen hiervon gewinnt.

4. Verfahren nach einem der voranstehenden Ansprüche, **dadurch gekennzeichnet, dass** man die Behandlung nach Anspruch 1b) mit einem Bisulfit (=Disulfit, Hydrogensulfit) durchführt.

5. Verfahren nach Anspruch 4, **dadurch gekennzeichnet, dass** die chemische Behandlung nach Einbetten der DNA in Agarose oder besonders bevorzugt nach Bindung der DNA an eine Oberfläche erfolgt.

6. Verfahren nach Anspruch 4, **dadurch gekennzeichnet, dass** bei der chemischen Behandlung ein die DNA-Duplex denaturierendes Reagenz und/oder ein Radikalfänger zugegen ist.

7. Verfahren nach einem der voranstehenden Ansprüche, **dadurch gekennzeichnet, dass** aus einer Probe genomische DNA extrahiert wird und dabei an eine Oberfläche gebunden wird.

8. Verfahren nach Anspruch 7, **dadurch gekennzeichnet, dass** diese Bindung durch Hybridisierung an ein immobilisiertes Oligonukleotid erfolgt.

9. Verfahren nach einem der voranstehenden Ansprüche, **dadurch gekennzeichnet, dass** die extrahierte DNA vor der Bindung durch Restriktionsenzyme gespalten wird.

10. Verfahren nach einem der voranstehenden Ansprüche, **dadurch gekennzeichnet, dass** man an die behandelte DNA mehrere verschiedene Oligonukleotide als Primer hybridisiert.

11. Verfahren nach einem der voranstehenden Ansprüche, **dadurch gekennzeichnet, dass** die markierten Nukleotide Guaninderivate sind, wobei diese in der Polymerasereaktion nur dann wesentlich eingebaut werden, wenn in der DNA Probe an den entsprechenden Positionen Cytosin-Methylierung vorlag.

12. Verfahren nach Anspruch 11. **dadurch gekennzeichnet, dass** das Ausmaß des Einbaus der Guaninbasen und damit auch die Anzahl der eingebauten Markierungen proportional zu der Methylierung in der zu untersuchenden DNA-Probe.

13. Verfahren nach einem der voranstehenden Ansprüche, **dadurch gekennzeichnet, dass** die Polymerasereaktion an der Position bevorzugt endet, die vor der DNA Isolierung durch den Einsatz einer Restriktionsendonuklease geschnitten wurde.

14. Verfahren nach einem der voranstehenden Ansprüche, **dadurch gekennzeichnet, dass** die in der Polymeraseraktion nicht eingebauten markierten Nukleotide durch Waschschritte entfernt werden und die DNA an eine Oberfläche gebunden vorliegt.

15. Verfahren nach einem der voranstehenden Ansprüche, **dadurch gekennzeichnet, dass** die Markierungen Fluoreszenzmarkierungen, Radionuklide, Chemiluminiszenmarkierungen oder abspaltbare Massenmarkierungen sind, welche in einem Massenspektrometer nachgewiesen werden.

16. Verfahren nach einem der voranstehenden Ansprüche, **dadurch gekennzeichnet, dass** die Markierungen indirekt durch Bindung eines wiederum anders markierten Antikörpers nachgewiesen werden.

17. Verfahren nach einem der voranstehenden Ansprüche, **dadurch gekennzeichnet dass** die behandelte DNA-Probe nach Schritt 1b) des Anspruchs 1 unter Verwendung von bevorzugt mindestens zwei Primeroligonukleotiden mittels einer Polymerasekettenreaktion amplifiziert wird.

18. Verfahren nach Anspruch 17, **dadurch gekennzeichnet, dass** die markierten Nukleotide Cytosinderivate sind, wobei diese in der Polymerasereaktion nur dann wesentlich eingebaut werden, wenn in der DNA Probe an den entsprechenden Positionen Cytosin-Methylierung vorlag.

19. Verfahren nach Anspruch 17, **dadurch gekennzeichnet, dass** die Amplifikation mehrerer Fragmente in einem Reaktionsgefäß in Form einer Multiplex-PCR durchgeführt wird.

20. Verfahren nach einem der voranstehenden Ansprüche, **dadurch gekennzeichnet, dass** der Methylierungsstatus von mehr als 10 Methylierungspositionen der zu analysierenden DNA in einem Experiment nachgewiesen wird.

21. Verfahren nach einem der voranstehenden Ansprüche, **dadurch gekennzeichnet, dass** bei mindestens einer der Amplifikationen einer der jeweiligen Primer an eine Festphase gebunden ist.

22. Verfahren nach einem der voranstehenden, **dadurch gekennzeichnet, dass** die Amplifikate insgesamt im Massenspektrometer nachgewiesen werden und somit durch ihre Masse eindeutig charakterisiert sind.

23. Verwendung eines Verfahrens nach einem der voranstehenden Ansprüche zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

24. Verwendung eines Verfahrens nach einem der voranstehenden Ansprüche zur Unterscheidung von Zelltypen oder Geweben oder zur Untersuchung der Zelldifferenzierung.

## Claims

1. A method for the analysis of the degree of methylation within a CpG-island in DNA samples is hereby **characterized in that** the following method steps are conducted:
a) DNA is extracted from a sample,
b) the DNA is treated, preferably with a bisulfite (= disulfite, hydrogen sulfite), in such a way that cytosine is converted into a base that is different in its base pairing behaviour in the DNA duplex, while 5-methylcytosine remains unchanged,
c) one or more oligonucleotide primers are hybridized to the treated DNA,
d) the hybridized primers are extended in a polymerase reaction, wherein labelled nucleotides are essentially incorporated only if cytosine bases were still present in the DNA treated according to step b) and wherein the degree of methylation is assumed as being similar and wherein the extent of the incorporation of labelled nucleotides correlates with the methylation in the DNA sample under investigation,
e) the labelled nucleotides that were not incorporated in the polymerase reaction are removed,
f) the number of labels in the fragment generated by primer extension is approximately determined by measuring the signal intensity emitted by these labels.

2. The method according to claim 1, further **characterized in that** the DNA samples are obtained from serum or other body fluids of an individual.

3. The method according to claim 1, further **characterized in that** the DNA samples are obtained from cell lines, blood, sputum, stool, urine, serum, cerebrospinal fluid, tissue embedded in paraffin, for example, tissue from eyes, intestine, kidney, brain, heart, prostate, lungs, breast or liver, histological slides and all possible combinations thereof.

4. The method according to one of the preceding claims, further **characterized in that** the treatment according to claim 1b} is conducted with a bisulfite (= disulfite, hydrogen sulfite).

5. The method according to claim 4, further **characterized in that** the chemical treatment is conducted after embedding the DNA in agarose or most preferably after binding the DNA to a surface.

6. The method according to claim 4, further **characterized in that** in the chemical treatment, a reagent that denatures the DNA duplex and/or a radical trap is present.

7. The method according to one of the preceding claims, further **characterized in that** genomic DNA is extracted from a sample and then is bound to a surface.

8. The method according to claim 7, further **characterized in that** this binding is performed by hybridization to an immobilized oligonucleotide.

9. The method according to one of the preceding claims, further **characterized in that** the extracted DNA is cleaved by restriction enzymes prior to the binding.

10. The method according to one of the preceding claims, further **characterized in that** several different oligonucleotides are hybridized as primers to the treated DNA.

11. The method according to one of the preceding claims, further **characterized in that** the labelled nucleotides are guanine derivatives, wherein these are essentially incorporated in the polymerase reaction, only if a cytosine methylation was present in the DNA sample at the corresponding positions.

12. The method according to claim 11, further **characterized in that** the extent of incorporation of guanine bases and thus also the number of incorporated labels is proportional to the methylation in the DNA sample under investigation.

13. The method according to one of the preceding claims, further **characterized in that** the polymerase reaction terminates preferably at the position which was cleaved by the use of a restriction endonuclease prior to the DNA isolation.

14. The method according to one of the preceding claims, further **characterized in that** the labelled nucleotides not incorporated in the polymerase reaction are removed by washing steps and the DNA is present bound to a surface.

15. The method according to one of the preceding claims, further **characterized in that** the labels are fluorescent labels, radionuclides, chemiluminescent labels or removable mass labels, which are detected in a mass spectrometer.

16. The method according to one of the preceding claims, further **characterized in that** the labels are indirectly detected by the binding of an antibody labelled in a different way.

17. The method according to one of the preceding claims, further **characterized in that** the DNA sample treated according to step 1b) of claim 1 is amplified with the use of preferably at least two primer oligonucleotides by means of a polymerase chain reaction.

18. The method according to claim 17, further **characterized in that** the labelled nucleotides are cytosine derivatives, wherein these are essentially incorporated in the polymerase reaction, only if a cytosine methylation was present in the DNA sample at the corresponding positions.

19. The method according to claim 17, further **characterized in that** the amplification of several fragments is conducted in one reaction vessel in the form of a multiplex PCR.

20. The method according to one of the preceding claims, further **characterized in that** the methylation state of more than 10 methylation positions of the DNA to be analyzed is detected in one experiment.

21. The method according to one of the preceding claims, further **characterized in that** in at least one of the amplifications, one of the respective primers is bound to a solid phase.

22. The method according to one of the preceding claims, further **characterized in that** the amplificates are detected are a whole in the mass spectrometer and are thus clearly **characterized by** their mass.

23. Use of a method according to one of the preceding claims for the diagnosis and/or prognosis of adverse events for patients or individuals, whereby these adverse events belong to at least one of the following categories: undesired drug effects; cancer diseases; CNS malfunctions, damage or disease; symptoms of aggression or behavioural disturbances; clinical, psychological and social consequences of brain damage; psychotic disturbances and personality disorders; dementia and/or associated syndromes; cardiovascular disease, malfunction and damage; malfunction, damage or disease of the gastrointestinal tract; damage or disease of the respiratory system; lesion, inflammation, infection, immunity and/or convalescence; malfunction, damage or disease of the body as a consequence of an abnormality in the development process; malfunction, damage or disease of the skin, the muscles, the connective tissue or the bones; endocrine and metabolic malfunction, damage or disease; headaches or sexual malfunction.

24. Use of a method according to one of the preceding claims for the differentiation of cell types or tissues or for the investigation of cell differentiation.

## Revendications

1. Procédé pour l'analyse du degré de méthylation à l'intérieur d'un îlot CPG dans des échantillons d'ADN, **caractérisé en ce qu'**on met en oeuvre les étapes suivantes :
a) on extrait l'ADN d'un échantillon,
b) on traite l'ADN, de préférence avec un bisulfite (= disulfite, hydrogénosulfite) de telle sorte que la cytosine soit convertie en une base différente du point de vue de son comportement en appariement de bases dans le duplex d'ADN, tandis que la 5-méthylcytosine reste inchangée,
c) on hybride à l'ADN traité une ou plusieurs amorces oligonucléotidiques,
d) on prolonge les amorces hybridées dans une réaction par polymérase, ce à l'occasion de quoi les nucléotides marqués ne sont pour l'essentiel incorporés que quand il existe encore des bases cytosines dans l'ADN traité après l'étape b), l'état de méthylation, dans les positions à étudier, étant supposé être analogue, et l'importance de l'incorporation des nucléotides marqués est corrélée à la méthylation dans l'échantillon d'ADN à étudier,
e) on élimine les nucléotides marqués qui n'ont pas été incorporés dans la réaction par polymérase,
f) on détermine approximativement le nombre des marqueurs dans le fragment produit par le prolongement d'amorce, en mesurant une intensité du signal émis par ces marqueurs.

2. Procédé selon la revendication 1, **caractérisé en ce qu'**on se procure les échantillons d'ADN à partir du sérum ou d'autres fluides corporels d'un individu.

3. Procédé selon la revendication 1, **caractérisé en ce qu'**on se procure les échantillons d'ADN à partir de lignées cellulaires, du sang, des crachats, des selles, de l'urine, du sérum, du liquide céphalorachidien, d'un tissu enrobé de paraffine, par exemple un tissu provenant des yeux, des intestins, des reins, du cerveau, du coeur, de la prostate, des poumons, du sein ou du foie, des porte-objets histologiques et de toutes les combinaisons possibles de ceux-ci.

4. Procédé selon l'une des revendications précédentes, **caractérisé en ce qu'**on met en oeuvre le traitement selon la revendication 1b) avec un bisulfite (= disulfite, hydrogénosulfite).

5. Procédé selon la revendication 4, **caractérisé en ce que** le traitement chimique est effectué après enrobage de l'ADN dans de l'agarose, ou d'une manière particulièrement préférée après liaison de l'ADN à une surface.

6. Procédé selon la revendication 4, **caractérisé en ce que**, lors du traitement chimique, on est en présence d'un réactif dénaturant le duplex d'ADN et/ou d'un capteur de radicaux.

7. Procédé selon l'une des revendications précédentes, **caractérisé en ce qu'**on extrait l'ADN génomique d'un échantillon, et on le lie à cette occasion à une surface.

8. Procédé selon la revendication 7, **caractérisé en ce que** cette liaison est effectuée par hybridation à un oligonucléotide immobilisé.

9. Procédé selon l'une des revendications précédentes, **caractérisé en ce que** l'ADN extrait est clivé par des enzymes de restriction avant la liaison.

10. Procédé selon l'une des revendications précédentes, **caractérisé en ce qu'**on hybride à l'ADN traité plusieurs oligonucléotides différents servant d'amorces.

11. Procédé selon l'une des revendications précédentes, **caractérisé en ce que** les nucléotides marqués sont des dérivés de la guanine, ces derniers n'étant essentiellement incorporés dans la réaction par polymérase que quand une méthylation de la cytosine était présente dans l'échantillon d'ADN sur les positions correspondantes.

12. Procédé selon la revendication 11, **caractérisé en ce que** l'importance de l'incorporation des bases guanines, et donc aussi le nombre des marqueurs incorporés, sont proportionnels à la méthylation dans l'échantillon d'ADN à étudier.

13. Procédé selon l'une des revendications précédentes, **caractérisé en ce que** la réaction par polymérase se termine de préférence sur la position qui avait été clivée, avant isolement de l'ADN, par utilisation d'une endonucléase de restriction.

14. Procédé selon l'une des revendications précédentes, **caractérisé en ce que** les nucléotides marqués non incorporés dans la réaction par polymérase sont éliminés par des étapes de lavage, l'ADN étant présent lié à une surface.

15. Procédé selon l'une des revendications précédentes, **caractérisé en ce que** les marqueurs sont des marqueurs par fluorescence, des radionucléotides, des marqueurs par chimioluminescence ou des marqueurs de masse éliminables, qui sont détectés dans un spectromètre de masse.

16. Procédé selon l'une des revendications précédentes, **caractérisé en ce que** les marqueurs sont détectés par liaison d'un anticorps qui, pour sa part, est marqué autrement.

17. Procédé selon l'une des revendications précédentes, **caractérisé en ce que** l'échantillon d'ADN traité est, après l'étape 1b) de la revendication 1, amplifié par utilisation de préférence d'au moins deux oligonucléotides amorces, à l'aide d'une réaction en chaîne par polymérase.

18. Procédé selon la revendication 17, **caractérisé en ce que** les nucléotides marqués sont des dérivés de la cytosine, ces derniers n'étant pour l'essentiel incorporés dans la réaction par polymérase que quand une méthylation de la cytosine était présente dans l'échantillon d'ADN sur les positions correspondantes.

19. Procédé selon la revendication 17, **caractérisé en ce que** l'amplification de plusieurs fragments est mise en oeuvre dans un réacteur sous forme d'une PCR multiplex.

20. Procédé selon l'une des revendications précédentes, **caractérisé en ce qu'**on détecte en une seule expérience l'état de méthylation de plus de 10 positions de méthylation de l'ADN à analyser.

21. Procédé selon l'une des revendications précédentes, **caractérisé en ce que** l'une des différentes amorces est liée à une phase solide lors d'au moins l'une des amplifications.

22. Procédé selon l'une des revendications précédentes, **caractérisé en ce que** les amplifiats sont globalement détectés au spectromètre de masse, et ainsi caractérisés d'une manière non ambiguë par leur masse.

23. Utilisation d'un procédé selon l'une des revendications précédentes pour le diagnostic et/ou le pronostic d'événements gênants pour des patients ou des individus, ces événements gênants appartenant au moins à l'une des catégories suivantes : les effets secondaires indésirables des médicaments ; les maladies cancéreuses ; les dysfonctionnements, lésions ou maladies du CNS ; les symptômes d'agression ou les troubles du comportement ; les conséquences cliniques, psychologiques et sociales des lésions cérébrales ; les troubles psychotiques et les troubles de la personnalité ; la démence et/ou les syndromes associés ; les maladies, dysfonctionnements et lésions cardiovasculaires ; les dysfonctionnements, lésions ou maladies du tractus gastro-intestinal ; les dysfonctionnements, lésions ou maladies du système respiratoire ; les lésions, inflammations, infections, problèmes immunitaires et/ou de reconvalescence ; les dysfonctionnements, lésions ou maladies du corps, en tant qu'aberrations lors du processus de développement ; les dysfonctionnements, lésions ou maladies de la peau, des muscles, du tissu conjonctif ou des os ; les dysfonctionnements, lésions ou maladies endocriniens et métaboliques ; les céphalées ou les dysfonctionnements sexuels.

24. Utilisation d'un procédé selon l'une des revendications précédentes pour distinguer des types cellulaires ou des tissus, ou pour étudier la différenciation cellulaire.
